(19) Europäisches Patentamt
European Patent Office
Office européen des brevets

(11) Numéro de publication : **0 312 412 B1**

# (12) FASCICULE DE BREVET EUROPEEN

(45) Date de publication du fascicule du brevet : **26.06.91 Bulletin 91/26**

(21) Numéro de dépôt : **88402388.8**

(22) Date de dépôt : **22.09.88**

(51) Int. Cl.$^5$ : **C07C 69/608,** C07C 57/26, C07C 33/14, C07F 7/18, C07C 233/10, C07C 255/31, C07D 309/10, C07D 295/02, C07C 43/162

## (54) Nouveaux dérivés du norbornane, leur procédé de préparation et compositions cosmétique et médicamenteuse les contenant.

(30) Priorité : **16.10.87 FR 8714298**

(43) Date de publication de la demande : **19.04.89 Bulletin 89/16**

(45) Mention de la délivrance du brevet : **26.06.91 Bulletin 91/26**

(84) Etats contractants désignés : **BE CH DE GB IT LI NL**

(56) Documents cités :
**EP-A- 0 000 719**
**EP-A- 0 001 553**
**GB-A- 2 122 200**
**US-A- 4 132 677**
**US-A- 4 267 111**
**CHEMICAL ABSTRACTS, volume 80, no. 23, 10 juin 1974, page 433, abstract no. 132887f; G.E. GREAM: "Synthesis of exo- and endo-2,3,3-trimethylnorbornane-2-carboxy lic acids"**

(73) Titulaire : **L'OREAL**
**14, Rue Royale**
**F-75008 Paris (FR)**

(72) Inventeur : **Solladie, Guy**
**4, rue d'Oslo**
**F-67000 Strasbourg (FR)**
Inventeur : **Forestier, Serge**
**14, Hameau des Lauriers**
**F-77410 Claye-Souilly (FR)**
Inventeur : **Lang, Gérard**
**44, avenue Lacour**
**F-95210 St-Gratien (FR)**

(74) Mandataire : **Peuscet, Jacques et al**
**Cabinet Peuscet 68, rue d'Hauteville**
**F-75010 Paris (FR)**

EP 0 312 412 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés du norbornane, leur procédé de préparation et leur utilisation en cosmétique ainsi qu'en médecine humaine et vétérinaire.

Ces dérivés du norbornane sont les composés de formule générale :

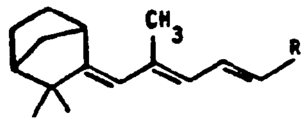 (I)

ou les sels correspondants, formule dans laquelle R représente soit un groupement de formule :

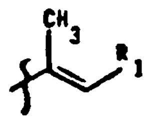 (II)

soit un groupement de formule :

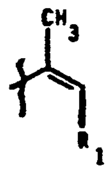 (III)

Dans les groupements de formule (II) et (III), $R_1$ représente un radical pris dans le groupe formé par les radicaux :

- oxazolinyle
- $-C\equiv N$
- $-CH_2-O-R_2$
- 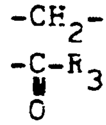

– $R_2$ représentant un atome d'hydrogène, un radical alkyle en $C_1$-$C_6$, un radical cyclopentyle, un radical cyclohexyle, un radical tétrahydropyrannyle, un radical aralkyle en $C_7$-$C_9$, éventuellement substitué par un ou plusieurs groupements alcoxy en $C_1$-$C_6$, un radical de formule :

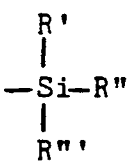 (IV)

dans laquelle R′, R″ et R‴, identiques ou différents, représentent un reste alkyle, linéaire ou ramifié, en $C_1$-$C_6$ ou un reste aryle éventuellement substitué par un radical alkyle en $C_1$-$C_4$

– $R_3$ représentant un atome d'hydrogène, le radical $-O-R_4$ ou le radical

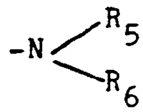
,

$R_5$ et $R_6$, identiques ou différents, représentant un atome d'hydrogène, un radical alkyle en $C_1$-$C_6$ ou, pris ensemble, formant un hétérocycle en $C_5$-$C_8$, $R_4$ représentant un atome d'hydrogène ou un radical alkyle en $C_1$-$C_6$.

Parmi les composés de formule (I) particulièrement préférés selon l'invention, on peut mentionner ceux possédant les structures suivantes :

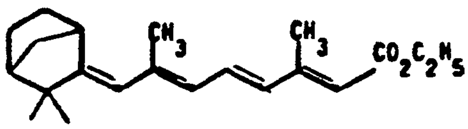
$(I_A)$

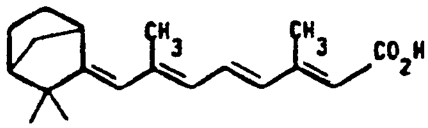
$(I_B)$

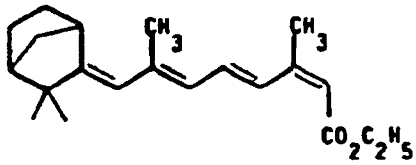
$(I_C)$
,

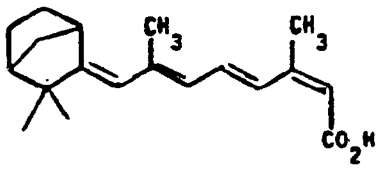
$(I_D)$

Les composés de formule (I) peuvent être synthétisés stéréospécifiquement selon la nature du substituant R.

Pour obtenir les composés de formule (I), on utilise un procédé caractérisé par le fait que, dans une première étape, on oxyde le dl-patchénol de formule (V) :

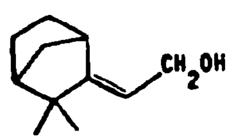
(V)

en dl-patchénal de formule (VI) :

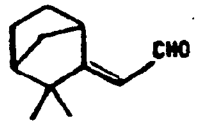
(VI)

par $MnO_2$ activé en milieu solvant ; dans une deuxième étape, on fait agir sur le composé de formule (VI) une

solution d'iodure de méthylmagnésium en milieu solvant et, après avoir stoppé la réaction, on oxyde lentement la solution par $MnO_2$ pour obtenir le composé de formule (VII) :

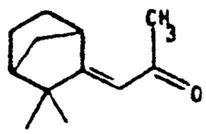

(VII)

dans une troisième étape, on prépare l'organomagnésien H-C≡C-MgBr en milieu solvant et on le fait agir sous courant d'acétylène sur le composé de formule (VII) pour obtenir le composé de formule (VIII) :

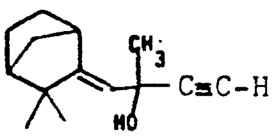

(VIII)

dans une quatrième étape, on fait agir en milieu solvant le bromure d'éthylmagnésium sur le composé de formule (VIII) puis on ajoute dans la solution du trans- ou du cis-formyl-3-butène-2-oate d'alkyl inférieur selon que l'on désire un composé de formule (I) dans lequel R représente un groupement de formule (II) ou (III) respectivement, afin d'obtenir respectivement un composé de formule (IX) ou (XVI) :

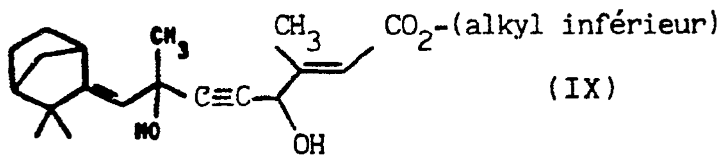

(IX)

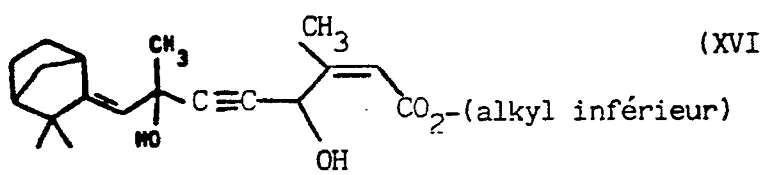

(XVI)

dans une cinquième étape, on fait agir, en milieu solvant, sur le composé ainsi obtenu, de l'hydrure de diisobutylaluminium (DIBAL) pour obtenir le triol de formule (X) ou (XVII) respectivement :

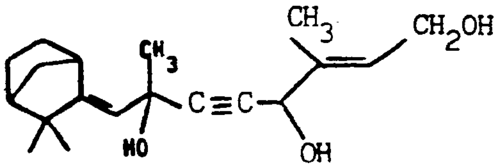

(X)

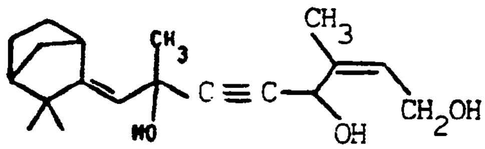

(XVII)

dans une sixième étape, on bloque la fonction OH en bout de chaine du triol obtenu en faisant agir sur ce composé un halogénure de tri-(alkyl ou aryl)silyle en présence d'imidazole pour obtenir le composé de formule (XI) ou (XVIII) respectivement :

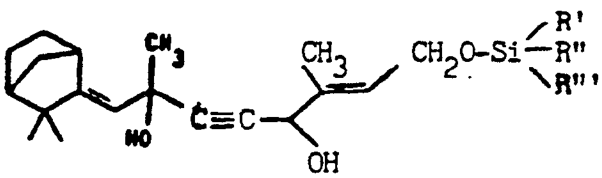

(XI)

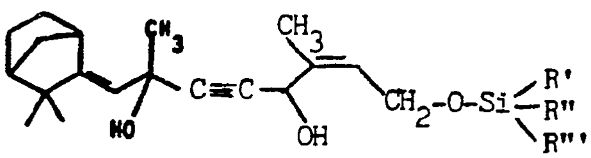

(XVIII)

où R', R", R‴ ont les significations précédemment indiquées, on réduit ce dernier composé en milieu solvant par l'hydrogène en présence d'un catalyseur Pd/$CaCO_3$ désactivé au plomb pour obtenir le diol de formule (XII) ou (XIX) respectivement :

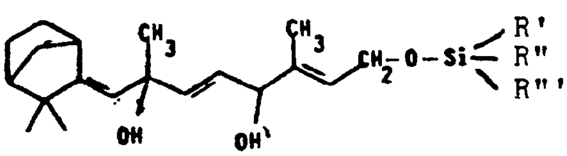

(XII)

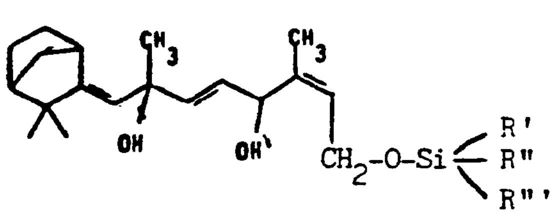

(XIX)

puis on élimine les deux fonctions OH du diol en faisant agir sur le composé obtenu, en milieu solvant anhydre, un mélange (trichlorure de titane, hydrure d'aluminium et de lithium) pour obtenir le composé de formule (XIII) ou (XX) respectivement :

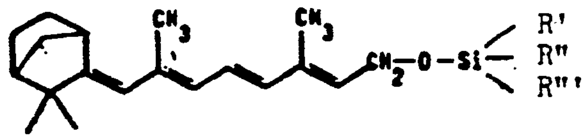

(XIII)

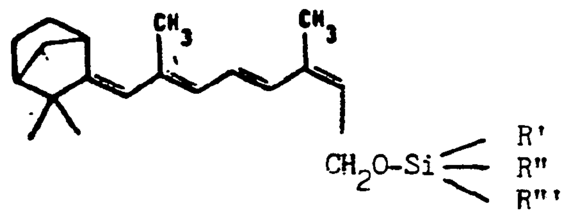

(XX)

et, dans une éventuelle septième étape, on transforme ce dernier composé pour obtenir un autre composé de formule (I) où R est un groupement de formule (II) ou (III) dans lequel $R_1$ est différent de

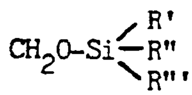 .

Dans un premier mode de réalisation destiné à préparer un composé de formule (I) dans lequel $R_1$ est un radical $CH_2OH$, on débloque l'extrémité de la chaîne du composé (XIII) ou (XX) par action en milieu solvant, d'au moins un fluorure pour obtenir le composé de formule (XIV) ou (XXI) respectivement :

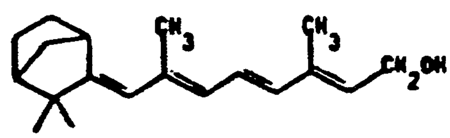

(XIV)

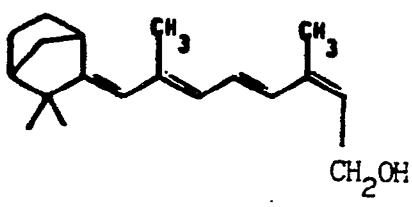

(XXI)

Dans un deuxième mode de réalisation destiné à préparer un composé dans lequel $R_1$ est un radical CHO, on utilise, comme composé de départ, le composé correspondant dans lequel $R_1=CH_2OH$, composé que l'on prépare comme ci-dessus, et on oxyde ce composé de formule (XIV) ou (XXI) en milieu solvant anhydre par action de $MnO_2$. On obtient ainsi les composés de formule (XV) ou (XXII) :

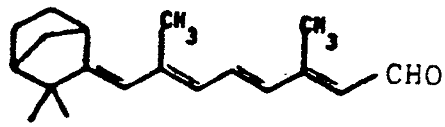

(XV)

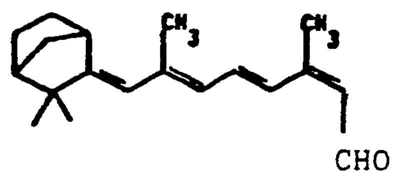

(XXII)

Lorsque l'on désire préparer un composé de formule (I), dans lequel $R_1$ est un radical $CO_2C_2H_5$, on utilise comme composé de départ un composé correspondant dans lequel $R_1$ est un radical CHO, composé que l'on prépare comme ci-dessus indiqué, et l'on soumet ce composé, à l'état dissous dans l'éthanol, à l'action d'un cyanure alcalin et de l'oxyde d'argent AgO. On obtient ainsi les composés ($I_A$) ou ($I_C$).

Lorsque l'on désire préparer un composé de formule (I) dans lequel $R_1$ est un radical $CO_2H$, on utilise comme composé de départ le produit correspondant dans lequel $R_1$ est un radical $CO_2C_2H_5$, ce produit ayant été préparé comme ci-dessus indiqué, et l'on soumet cet ester à l'action d'une solution hydroalcoolique d'une base forte, après quoi on ramène la solution à un pH acide. On obtient ainsi les composés ($I_B$) ou ($I_D$).

Ces composés sont utilisables :

1) pour traiter les affections dermatologiques liées à un désordre de la kératinisation portant sur la différenciation et sur la prolifération notamment pour traiter les acnés vulgaires, comédoniennes, polymorphes, les acnés nodulo kystiques, conglobata, les acnés séniles, les acnés secondaires telles que l'acné solaire, médicamenteuse, professionnelle ;

2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen ;

3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriasique, ou encore l'atopie cutanée, telle que l'eczéma, ou l'atopie respiratoire ;

4) pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les proliférations pouvant également être induites par les ultra-violets notamment dans le cas des épithelioma baso et spino cellulaires ;

5) pour traiter d'autres désordres dermatologiques tels que les maladies bulleuses et les maladies du collagène ;

6) pour des traitements dans le domaine ophtalmologique, notamment pour les cornéopathies ;

7) pour lutter contre le vieillissement de la peau, en particulier sous l'effet du soleil.

Les composés selon l'invention présentent une bonne activité comédolytique dans le test décrit par BONNE et al. dans "International Journal of Cosmetic Sience, 3, 23-28 (1981)", cette expérimentation étant effectuée sur la peau de souris hairless rhino, comme préconisé par Van Scott en 1972.

La présente invention a donc aussi pour objet une nouvelle composition médicamenteuse, destinée notamment au traitement des affections susmentionnées, caractérisée par le fait qu'elle comporte, dans un support pharmaceutiquement acceptable, au moins un composé de formule (I) ou un sel correspondant.

Comme support des compositions, on peut utiliser tout support conventionnel, le composé actif se trouvant soit à l'état dissous, soit à l'état dispersé dans ledit support.

L'administration peut être effectuée par voie entérale, parentérale, rectale, topique ou oculaire. Les composés selon l'invention sont généralement administrés à une dose journalière d'environ 0,02 µg/kg à 2 mg/kg de poids corporel.

Pour la voie entérale, les compositions selon l'invention peuvent se présenter sous forme de comprimés, de gélules, de dragées, de sirops, de suspensions, de solutions, de poudres, de granulés, d'émulsions.

Pour la voie parentérale, les compositions selon l'invention peuvent se présenter sous forme de solutions

ou suspensions pour perfusions ou pour injections.

Pour la voie topique, les compositions selon l'invention peuvent se présenter sous forme d'onguents, de teintures, de crèmes, de pommades, de poudres, de timbres, de tampons imbibés, de solutions, d'émulsions, de lotions, de gels, de sprays ou encore de suspensions. Ces compositions peuvent être soit sous forme anhydre, soit sous forme aqueuse selon l'indication clinique. On observe une bonne activité de ces composés par voie topique sur une très grande gamme de dilution ; on peut utiliser, notamment, des concentrations en produit actif allant de 0,0001 à 5% en poids par rapport au poids total de la composition ; il est bien entendu possible d'utiliser des concentrations supérieures si nécessaire pour une application thérapeutique particulière ; toutefois, les concentrations préférées en produit actif sont comprises entre 0,001 et 1% en poids par rapport au poids total de la composition.

Par voie oculaire, les compositions selon l'invention se présentent, de préférence, sous forme de collyres.

Les composés de formule (I) trouvent également, selon l'invention, une application dans le domaine cosmétique, en particulier dans l'hygiène corporelle et capillaire et, notamment, pour les peaux à tendance acnéique, pour la repousse des cheveux, pour combattre la chute des cheveux, pour lutter contre l'aspect gras de la peau ou des cheveux, pour prévenir ou traiter les effets néfastes du soleil ou pour le traitement des peaux physiologiquement sèches.

La présente invention a donc également pour objet une composition cosmétique contenant, dans un support cosmétiquement acceptable, au moins un composé de formule (I) ou un sel correspondant. Cette composition peut se présenter, notamment, sous forme de lotion, gel, savon ou shampooing.

La concentration en composé(s) de formule (I), dans les compositions cosmétiques selon l'invention, est comprise entre 0,0001 et 2% en poids et, de préférence, entre 0,001 et 1% en poids par rapport au poids total de la composition.

Les compositions médicamenteuse et cosmétique selon l'invention peuvent contenir des additifs inertes ou pharmacodynamiquement ou cosmétiquement actifs et, notamment : des agents hydratants comme la thiamorpholinone et ses dérivés ou l'urée ; des agents antiséborrhéiques, tels que la S-carboxyméthylcystéine, la S-benzyl-cystéamine et leurs dérivés, la tioxolone ; des agents anti-acnéiques comme le peroxyde de benzoyle ; des antibiotiques comme l'érythromycine et ses esters, la néomycine, les tétracyclines ou les polyméthylène-4,5 isothiazolinones-3 ; des agents favorisant la repousse des cheveux, comme le "Minoxidil" (diamino-2,4-pipéridino-6-pyrimidine oxyde-3) et ses dérivés, le "Diazoxide" (chlorométhyl-3 benzothiadiazine-1,2,4 dioxyde-1,1) et le "Phénytoïn" (diphényl-5,5 imidazolinedione-2,4) ou encore l'iodure d'oxapropanium ; des agents anti-inflammatoires stéroïdiens ou non stéroïdiens ; des caroténoïdes et, notamment, le β-carotène; des agents anti-psoriasiques tels que l'anthraline et ses dérivés et les acides eicosatétraynoïque-5,8,11,14 et triynoïque-5,8,11 et leurs esters et amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, de anti-oxydants tels que l'α-tocophérol, le butylhydroxy-anisole ou le butylhydroxy-toluène.

On va donner maintenant, à titre purement illustratif et non limitatif, plusieurs exemples de préparation des composés actifs de formule (I) ainsi que plusieurs exemples de compositions les contenant.

EXEMPLE 1 :

Préparation du composé de formule (XIII) dans laquelle R'=R"=$CH_3$ et R'''=tbutyle, ce composé répondant à la formule (I) où R est un groupement de formule (II) dans lequel $R_1$=$CH_2O$-$R_2$ avec

$R_2$ = 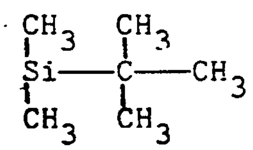

1ère étape : préparation du composé de formule (VI) :

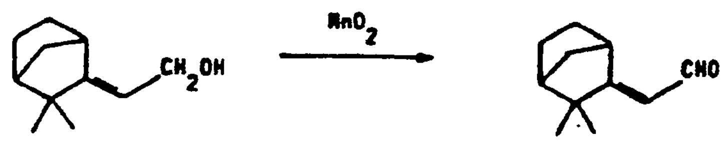


A une solution de 50 g (0,3 mole) de dl-patchénol dans un litre de dichlorométhane, sont ajoutés en une seule fois 10 équivalents de $MnO_2$ activé (préparés selon la méthode décrite par J. ATTENBURROW, J. Chem. Soc. 1952, 1104). Le mélange est agité à température ambiante pendant une nuit puis filtré et le solvant est évaporé sous pression réduite. On obtient ainsi 49 g de dl-patchénal.

2ème étape : préparation du composé de formule (VII) :

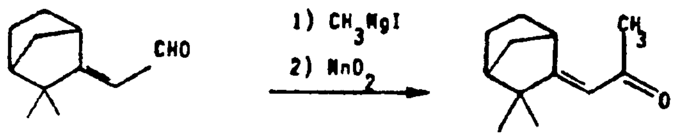


4 g (24,4 mmoles) de patchénal en solution dans 50 $cm^3$ d'éther anhydre sont ajoutés au goutte à goutte à une solution d'iodure de méthylmagnésium (1,5 éq., préparé à partir de 5,2 g (36,6 mmoles) d'iodure de méthyle et 0,9 g de magnésium dans 50 $cm^3$ d'éther). Le mélange réactionnel est agité pendant 15 mn à température ambiante puis versé sur une solution saturée froide de chlorure d'ammonium. La phase aqueuse est extraite trois fois avec de l'éther. Les phases organiques sont ensuite lavées avec une solution saturée de chlorure de sodium et séchées sur sulfate de magnésium. La solution éthérée est ensuite rapidement filtrée sur de la silice et le filtrat est immédiatement oxydé par 10 équivalents de $MnO_2$. Le milieu réactionnel est agité pendant trois jours à température ambiante, puis filtré et le solvant est distillé sous pression réduite. On obtient ainsi 3,7 g de produit attendu (rendement = 85%), qui est utilisé sans autre purification pour la troisième étape.

3ème étape : préparation du composé de formule (VIII) :

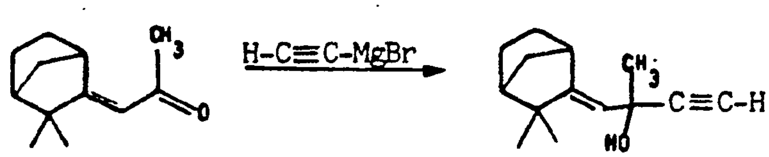


Un courant d'acétylène est envoyé lentement dans 400 $cm^3$ de tétrahydrofuranne anhydre refroidi à −40°C pendant 1 heure. Le bain réfrigérant est retiré et une solution de bromure d'éthylmagnésium (préparée à partir de 0,2 mole, 21,8 g de bromure d'éthyle, 4,9 g de magnésium et 100 $cm^3$ de tétrahydrofuranne anhydre) est rapidement ajoutée à la solution d'acétylène en maintenant la température au dessous de 35°C. Après l'addition, le milieu réactionnel est agité pendant 30 mn à température ambiante. On ajoute ensuite lentement 23,2 g (0,13 mole) de composé obtenu au cours de la deuxième étape en solution dans 100 $cm^3$ de tétrahydrofuranne. L'agitation est poursuivie pendant une heure. Le courant d'acétylène, qui avait été maintenu jusqu'alors, est arrêté et le milieu réactionnel est versé sur une solution saturée de chlorure d'ammonium. La phase aqueuse est extraite deux fois à l'éther. Les phases organiques sont lavées avec une solution saturée de chlorure de sodium, séchées sur sulfate de magnésium et le solvant est distillé sous pression réduite.

Le produit obtenu est purifié par chromatographie sur gel de silice en utilisant comme solvant un mélange 95/5 (en volumes) d'hexane et d'acétate d'éthyle. On obtient ainsi 23,9 g (rendement = 90%) de produit attendu.

4ème étape : préparation du composé de formule (IX) :

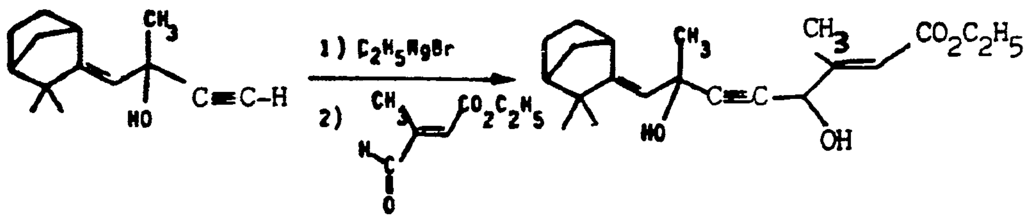


Le composé obtenu au cours de la 3ème étape (1,3 g, soit 6,36 mmoles) est ajouté à une solution de bromure d'éthylmagnésium (13,4 mmoles) dans 40 cm³ de tétrahydrofuranne. Le milieu réactionnel est agité pendant une heure puis on ajoute 0,9 g (6,36 mmoles) de transformyl-3-butène-2-oate d'éthyle. Après 15 minutes, le mélange est versé sur une quantité équivalente de chlorure d'ammonium saturé. Après extraction à l'éther, les phases organiques sont séchées sur sulfate de sodium et le solvant est distillé sous pression réduite.

Le produit est purifié par chromatographie sur gel de silice en utilisant comme solvant un mélange 30/70 (en volumes) d'acétate d'éthyle et d'hexane.

Le produit attendu est obtenu avec un rendement de 75%.

5ème étape : préparation du composé de formule (X) :

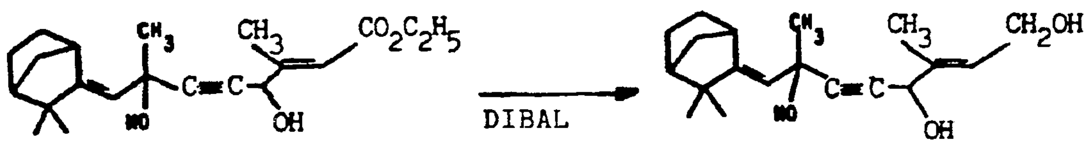


On refroidit à −20°C une solution de 1,4 g (4,3 mmoles) de composé obtenu dans la 4ème étape dans 100 cm³ de tétrahydrofuranne anhydre. On ajoute rapidement 11,5 cm³ d'une solution 1,5 M d'hydrure de diisobutylaluminium (DIBAL) (17,2 mmoles). Dix minutes après la fin de l'addition, on ajoute une solution saturée de chlorure d'ammonium puis une solution à 20% d'acide chlorhydrique pour dissoudre le précipité formé. Après séparation des phases, la phase aqueuse est extraite deux fois à l'éther. Les phases organiques sont lavées par une solution saturée de chlorure de sodium, séchées sur sulfate de sodium et le solvant est distillé sous pression réduite.

Le produit attendu est obtenu avec un rendement de 90%. Il est utilisé sans autre purification pour l'étape suivante.

6ème étape : préparation du composé de formule (XIII) :

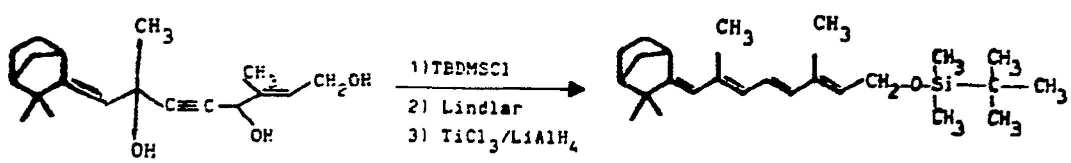


a) préparation du composé de formule (XI)

A 3,02 g du triol obtenu au cours de la 5ème étape dans 150 cm³ de diméthylformamide sont ajoutés un équivalent (1,5 g) de chlorure de tertiobutyl-diméthylsilyle et 2 équivalents (1,36 g) d'imidazole. La solution est agitée pendant 6 heures à température ambiante puis diluée par 1 litre d'eau. Le produit est extrait à l'éther (3 fois 120 cm³). Les phases éthérées sont lavées par une solution saturée de chlorure d'ammonium. Après séchage sur sulfate de magnésium et distillation du solvant sous pression réduite, on obtient le produit attendu sous forme d'une huile, qui est utilisée sans autre purification dans le stade suivant.

b) préparation du composé de formule (XII) :

On dissout 0,42 g de composé obtenu ci-dessus dans 20 $cm^3$ de méthanol absolu. Après addition de 0,8 g de catalyseur de Lindlar ($Pd/CaCO_3$ désactivé au plomb) l'air contenu dans le réacteur est éliminé et remplacé par de l'hydrogène. La suspension est alors agitée vigoureusement pendant 72 heures et la réaction est suivie en chromatographie sur couche mince de silice en utilisant un mélange acétate d'éthyle/hexane (80/20 en volumes) comme éluant.

Le catalyseur est filtré et le solvant est évaporé. Le produit ainsi obtenu est utilisé sans purification pour le stade suivant.

c) préparation du composé de formule (XIII) :

Deux équivalents (1,43 g) de $TiCl_3$ sont pesés dans un ballon préalablement séché à l'étuve. 30 $cm^3$ de tétrahydrofuranne anhydre sont ajoutés et un équivalent de $LiAlH_4$ dosé en solution dans l'éther (0,176 g) est introduit sous argon.

Le mélange est alors agité pendant 10 minutes à température ambiante et on ajoute 0,8 équivalent (1,56 g) du diol obtenu ci-dessus en solution dans 20 $cm^3$ de tétrahydrofuranne. Après une heure à température ambiante, le milieu est traité par 10 $cm^3$ d'eau et 60 $cm^3$ d'acide chlorhydrique 0,1 N.

Les phases sont séparées et la phase aqueuse est extraite par deux fois 30 $cm^3$ d'éther. Les phases organiques sont ensuite lavées par 60 $cm^3$ d'une solution saturée de chlorure de sodium puis séchées sur sulfate de magnésium. Le solvant est distillé sous pression réduite. On obtient ainsi 1,30 g de produit de formule (XIII).

EXEMPLE 2 :

Préparation du composé de formule (XIV) constituant un composé de formule (I) où R est un groupement de formule (II) dans lequel $R_1=CH_2OR_2$ avec $R_2=H$

1 g de composé obtenu à l'exemple 1 est dissous dans 15 $cm^3$ d'un mélange de tétrahydrofuranne et d'acétonitrile contenant 1/3 en volume de tétrahydrofuranne. La solution ainsi obtenue est traitée par 5 équivalents de fluorure de sodium anhydre et 0,1 équivalent de fluorure de pyridinium pur parfaitement sec et broyé en fines particules juste avant l'emploi. La réaction est effectuée à l'abri de la lumière, sous argon, après avoir ajouté à la solution 2 g de tamis moléculaire 4 Å.

Après 5 heures d'agitation à température ambiante, le milieu réactionnel est traité par 10 $cm^3$ d'une solution saturée de chlorure d'ammonium et 10 $cm^3$ d'eau. Après décantation, la phase aqueuse est extraite deux fois par 10 $cm^3$ d'éther. Les phases organiques sont combinées et lavées par 20 $cm^3$ d'une solution saturée en chlorure de sodium. Après séchage sur sulfate de sodium, le solvant est distillé sous pression réduite à température ambiante. On obtient ainsi 0,60 g de produit de formule (XIV).

EXEMPLE 3 :

Préparation du composé de formule (XV) constituant un composé de formule (I) où R est un groupement de formule (II) dans lequel $R_1 = \underset{\displaystyle O}{\overset{\|}{C}}-R_3$ avec $R_3 = H$

On dissout 1 g du composé obtenu à la fin de l'exemple 2 (sans purification complémentaire) dans 15 $cm^3$ de tétrachlorure de carbone anhydre à l'abri de la lumière. On ajoute, sous agitation vigoureuse, 1,5 g (5 équivalents) de $MnO_2$ fraîchement préparé. Après 30 minutes d'agitation à température ambiante, la suspension est filtrée rapidement sur gel de silice et le solvant est distillé à température ambiante sous pression réduite. On obtient 0,90 g de produit de formule (XV).

EXEMPLE 4 :

Préparation du composé de formule ($I_A$)

On dissout 1 g du composé obtenu à la fin de l'exemple 3 (sans purification complémentaire) dans 15 $cm^3$ d'éthanol. On ajoute successivement 5 équivalents de cyanure de sodium (0,86 g) et 6 équivalents de AgO (2,6 g). La suspension ainsi obtenue est agitée à 40°C à l'abri de la lumière pendant 14 heures. Le milieu réactionnel est filtré et la solution est chromatographiée sur gel de silice (éluant acétate d'éthyle/hexane contenant 5% en volumes d'acétate d'éthyle).

Après évaporation du solvant sous pression réduite, on obtient le produit attendu possédant les caractéristiques suivantes en RMN (résonance magnétique nucléaire) : Spectre de RMN $^1H$ en solution dans $CDCl_3$:

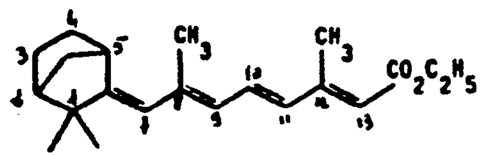

δ = 1,05-1,06 ppm (2s, 6H, 2 $CH_3$ en 1)
δ = 1,24-1,74 ppm (m 6H, $CH_2$ du cycle)
δ = 1,29 ppm (t, 3H, J=7Hz, $CH_3$ du groupe éthyle)
δ = 1,90 ppm (s large, 1H, H en 2)
δ = 2,04 ppm (s, 3H, $CH_3$ en 8)
δ = 2,35 ppm (s, 3H, $CH_3$ en 12)
δ = 3,31 ppm (s large, 1H, H en 5)
δ = 4,16 ppm (q, 2H, J=7Hz, $CH_2$ du groupe éthyle)
δ = 5,61 ppm (s, 1H, H en 13)
δ = 5,75 ppm (s, 1H, H en 7)
δ = 6,08 ppm (d, 1H, J=11Hz, H en 9)
δ = 6,21 ppm (d, 1H, J=15Hz, H en 11)
δ = 6,93 ppm (dd, 1H, J=11Hz et 15Hz, H en 10)

## EXEMPLE 5

### Préparation du composé de formule ($I_B$)

On met en suspension 0,5 g de l'ester obtenu à l'exemple 4 dans 25 $cm^3$ d'éthanol et 25 $cm^3$ de solution aqueuse 6 M de soude. On agite à 50°C à l'abri de la lumière jusqu'à dissolution complète.

Le milieu réactionnel est alors dilué par 500 $cm^3$ d'eau et l'éthanol est distillé sous pression réduite. La phase aqueuse est acidifiée jusqu'à pH 3 par addition d'acide chlorhydrique en solution aqueuse à 10% en poids. On extrait par 3 fois 50 $cm^3$ d'éther. La phase organique est séchée sur sulfate de sodium et le solvant est distillé sous pression réduite. On obtient le produit attendu après recristallisation dans le méthanol. Ce produit a les caractéristiques suivantes :
Point de fusion : 173-175°C
Spectre de RMN $^1H$ en solution dans $CDCl_3$ :

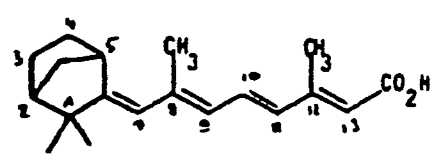

δ = 1,05-1,06 ppm (2s, 6H, 2 $CH_3$ en 1)
δ = 1,22-1,93 ppm (m 7H, $CH_2$ du cycle et H en 2)
δ = 2,03 ppm (s, 3H, $CH_3$ en 8)
δ = 2,36 ppm (s, 3H, $CH_3$ en 12)
δ = 3,32 ppm (s large, 1H, H en 5)
δ = 5,61 ppm (s, 1H, H en 13)
δ = 5,78 ppm (s, 1H, H en 7)
δ = 6,15 ppm (d,1H, J=11Hz, H en 9)
δ = 6,26 ppm (d, 1H, J=16Hz, H en 11)
δ = 6,98 ppm (dd, 1H, J=11Hz et 16Hz, H en 10)

EXEMPLE 6

Préparation du composé de formule (XX) dans laquelle R′=R″=R‴=$C_2H_5$, ce composé correspondant à la formule (I) où R est un groupement de formule (III) dans lequel $R_1$=$CH_2$-O-$R_2$ avec

$R_2$ =
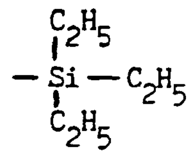

On reproduit à l'identique les trois premières étapes de l'exemple 1.

4ème étape : préparation du composé de formule (XVI)

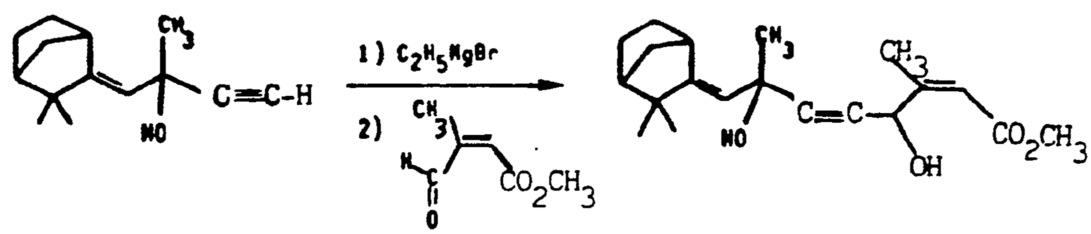


Ce composé est obtenu selon le mode opératoire décrit dans la 4ème étape de l'exemple 1 mais le trans-formyl-3-butène-2-oate d'éthyle est remplacé par le cis-formyl-3-butène-2-oate de méthyle.

Le produit attendu est obtenu avec un rendement de 70% après chromatographie sur gel de silice en utilisant comme éluant un mélange d'acétate d'éthyle et d'hexane (30/70 en volumes).

5ème étape : préparation du composé de formule (XVII) :

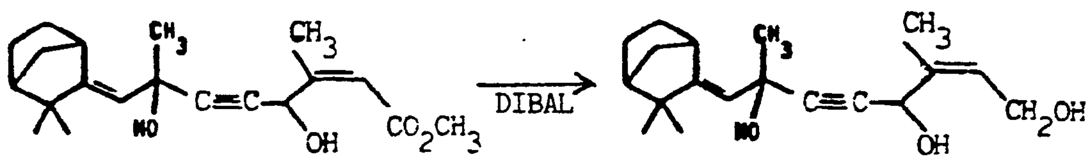


Ce composé est obtenu selon le mode opératoire décrit dans la 5ème étape de l'exemple 1 en utilisant l'isomère 12-cis obtenu au cours de l'étape précédente.

6ème étape : préparation du composé de formule (XX) :

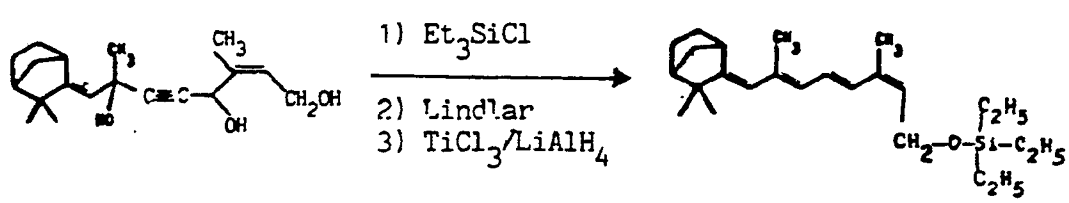


a) préparation du composé de formule

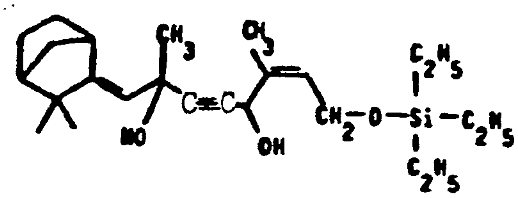

Ce composé est obtenu selon le mode opératoire décrit au premier stade de la 6ème étape de l'exemple 1. Le chlorure de tertiobutyldiméthylsilyle est remplacé par le chlorure de triéthylsilyle et le triol 12-trans est remplacé par le triol 12-cis obtenu à la fin de la 5ème étape de l'exemple 6.

b) préparation du composé de formule (XX) :

Ce composé est obtenu selon le mode opératoire décrit dans les 2ème et 3ème stades de la 6ème étape de l'exemple 1 : l'isomère 12-trans est remplacé par l'isomère 12-cis à chaque stade.

EXEMPLE 7 :

Préparation d'un composé de formule (XXI),constituant un composé de formule (I) où R est un groupement de formule (III) dans lequel $R_1=CH_2OR_2$ avec $R_2=H$

Ce composé est obtenu selon le mode opératoire décrit à l'exemple 2 en utilisant le produit de formule (XX) obtenu à l'exemple 6 à la place du produit obtenu à la fin de l'exemple 1.

EXEMPLE 8 :

Préparation d'un composé de formule (XXII), constituant un composé de formule (I) où R est un groupement de formule (III) dans lequel $R_1 = \underset{\underset{O}{\|}}{C}-R_3$ avec $R_3=H$

Ce composé est obtenu selon le mode opératoire décrit dans l'exemple 3 en remplaçant l'isomère 12-trans par l'isomère 12-cis obtenu à la fin de l'exemple 7.

EXEMPLE 9 :

Préparation du composé de formule ($I_C$)

Ce composé est obtenu selon le mode opératoire décrit dans l'exemple 4 en remplaçant l'isomère 12-trans par l'isomère 12-cis obtenu à l'exemple 8.

Après chromatographie sur gel de silice en utilisant un mélange d'acétate d'éthyle et d'hexane contenant 5% d'acétate d'éthyle, on obtient le produit attendu possédant les caractéristiques suivantes en RMN :

Spectre de RMN $^1H$ en solution dans $CDCl_3$

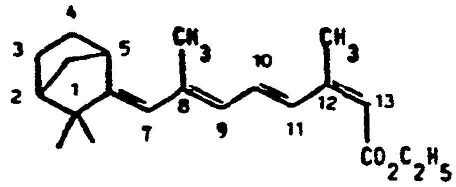

δ = 1,05-1,06 ppm (2s, 6H, 2 $CH_3$ en 1)

δ = 1,26-1,76 ppm (m 6H, $CH_2$ du cycle)

δ = 1,29 ppm (T, 3H, $CH_3$ du groupe éthyle, J=7Hz)

δ = 1,91 ppm (s large, 1H, H en 2)
δ = 2,03 ppm (s, 3H, $CH_3$ en 8)
δ = 2,06 ppm (s, 3H, $CH_3$ en 12)
δ = 3,30 ppm (s large, 1H, H en 5)
δ = 4,17 ppm (q, 2H, J=7Hz, $CH_2$ du groupe éthyle)
δ = 5,61 ppm (s, 2H, H en 13 et H en 7)
δ = 6,18 ppm (d, 1H, J=11Hz, H en 9)
δ = 6,92 ppm (dd, 1H, J=11 et 15Hz, H en 10)
δ = 7,72 ppm (d, 1H, J=15Hz, H en 11)

EXEMPLE 10 :

Préparation du composé de formule ($I_D$) :

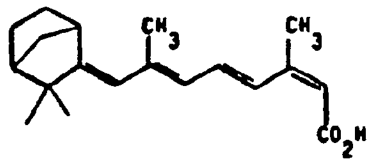

Ce produit est obtenu selon le mode opératoire décrit à l'exemple 5 ; l'isomère 12-trans est remplacé par l'isomère 12-cis obtenu à l'exemple 9. Après recristallisation dans l'éther puis dans le méthanol, on obtient le produit attendu possédant les caractéristiques suivantes :
Spectre de RMN $^1H$ en solution dans $CDCl_3$

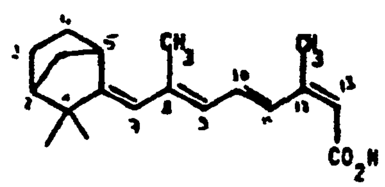

δ = 1,05-1,06 ppm (2s, 6H, 2 $CH_3$ en 1)
δ = 1,18-1,76 ppm (m 6H, $CH_2$ du cycle)
δ = 1,91 ppm (pic large, 1H, H en 2)
δ = 2,05 ppm (s, 3H, $CH_3$ en 8)
δ = 2,09 ppm (s, 3H, $CH_3$ en 12)
δ = 3,22 ppm (pic large, 1H, H en 5)
δ = 5,64 ppm (s, 2H, H en 13 et H en 7)
δ = 6,21 ppm (d, 1H, J=11, 5Hz, H en 9)
δ = 6,96 ppm (dd, 1H, J=11,5 et 15Hz, H en 10)
δ = 7,70 ppm (d, 1H, J=15Hz, H en 11)
Point de fusion : 181-183°C (décomposition)

EXEMPLE 11 :

On prépare un gel en réalisant la formulation suivante :

| | | |
|---|---|---|
| - composé de formule $I_B$ ................ | 0,050 | g |
| - Erythromycine base .................... | 4,000 | g |
| - Butylhydroxytoluène .................. | 0,050 | g |
| - Hydroxypropylcellulose vendu par la société HERCULES sous le nom de "KLUCEL HF" ....................... | 2,000 | g |
| - Ethanol (à 95°) .............qsp..... | 100,000 | g |

Ce gel est appliqué sur une peau à dermatose ou une peau acnéique 1 à 3 fois par jour. Après 6 à 12 semaines de traitement à raison de 2 à 10 mg par cm² de peau traitée et par application, on observe une amélioration significative.

EXEMPLE 12 :

On prépare la formulation suivante destinée à être conditionnée dans une gélule :

| | | |
|---|---|---|
| - composé de formule $I_A$ ................ | 0,060 | g |
| - Amidon de maïs ...................... | 0,060 | g |
| - Lactose ..................qsp......... | 0,300 | g |

Les gélules utilisées sont constituées de gélatine, d'oxyde de titane et d'un conservateur.

On administre à un individu adulte 1 à 3 gélules par jour dans le traitement du psoriasis. Après 30 jours de traitement à raison de 1 mg par kg de sujet traité et par jour, on observe une amélioration significative.

EXEMPLE 13 :

On prépare une lotion antiséborrhéique en procédant au mélange des ingrédients suivants :

| | | |
|---|---|---|
| - composé de formule $I_B$ .................. | 0,030 | g |
| - Propylène glycol ........................ | 5,000 | g |
| - Butylhydroxytoluène ..................... | 0,100 | g |
| - Ethanol ..................qsp............ | 100,000 | g |

Cette lotion est appliquée deux fois par jour sur un cuir chevelu séborrhéique. Après 2 à 6 semaines de traitement à raison de 1 à 4 mg par cm² traité et par application, on observe une amélioration significative.

EXEMPLE 14 :

On prépare un gel pour application topique en procédant au mélange des ingrédients suivants :

- composé de formule $I_B$ ............... 0,05 g
- Ethanol ............................... 43,00 g
- α-tocophérol ......................... 0,05 g
- Acide polyacrylique réticulé vendu par la société "GOODRICH" sous la dénomination "CARBOPOL 940" .............. 0,50 g
- Triéthanolamine (solution aqueuse à 20 %) .............................. 3,80 g
- Eau .................................... 9,30 g
- Propylène glycol .........qsp......... 100,00 g

Ce gel est appliqué deux fois par jour sur une peau acnéique. Après 6 à 12 semaines de traitement à raison de 2 à 10 mg par cm² de peau traitée et par application, on observe une amélioration significative.

EXEMPLE 15 :

On prépare un comprimé non soluble en procédant au mélange des substances suivantes :

- composé de formule $I_D$ ................. 0,025 g
- Lactose ................................ 0,082 g
- Acide stéarique ........................ 0,003 g
- Talc purifié ........................... 0,015 g
- Edulcorant ..................qs
- Colorants ...................qs
- Amidon de riz ...............qsp........ 0,500 g

On administre trois comprimés par jour par voie orale à un individu atteint de psoriasis. Après 30 jours de traitement à raison de 1 mg par kg de sujet traité et par jour, on observe une amélioration significative.

EXEMPLE 16 :

On prépare une solution en réalisant la formulation suivante :

- composé de formule $I_C$ .................. 0,20 g
- Polyéthylèneglycol (PM = 400) .......... 80,00 g
- Ethanol (à 95 °) ............qsp........100,00 g

On applique cette solution sur une peau acnéique 3 fois par jour. Après 6 à 12 semaines de traitement à raison de 1 à 4 mg par cm² de peau traitée et par application, on observe une amélioration significative.

EXEMPLE 17 :

On prépare une lotion capillaire contre la chute et pour la repousse des cheveux en procédant au mélange des ingrédients suivants :

- Propylène glycol.......................... 20,00 g
- Ethanol................................... 34,92 g
- Polyéthylène glycol (PM = 400)......... 40,00 g
- Eau........................................ 4,00 g
- Butylhydroxyanisole................... 0,01 g
- Butylhydroxytoluène................... 0,02 g
- composé de formule $I_B$.................. 0,05 g
- Diamino-2,4 pipéridino-6 pyrimidine oxyde-3... 1,00 g

On applique cette lotion 2 fois par jour sur un cuir chevelu ayant subi une chute des cheveux importante. Après 3 mois de traitement à raison de 1 ml par application, on observe une amélioration significative.

EXEMPLE 18 :

On réalise un kit anti-acné comprenant deux parties :
a) on prépare un gel ayant la formulation suivante :

- Alcool éthylique.................. 48,40 g
- Propylène glycol.................. 50,00 g
- Acide polyacrylique réticulé vendu par la société"GOODRICH"sous la dénomination "CARBOPOL 940"....... 1,00 g
- Butylhydroxyanisole.............. 0,05 g
- Butylhydroxytoluène.............. 0,05 g
- α-tocophérol.................... 0,10 g
- composé de formule $I_B$............ 0,10 g

b) on prépare un gel ayant la formulation suivante :

- Alcool éthylique.................. 5,00 g
- Propylène glycol.................. 5,00 g
- Sel disodique de l'acide éthylène diamine tétracétique.............. 0,05 g
- Acide polyacrylique réticulé vendu par la société GOODRICH sous la dénomination "CARBOPOL 940"...... 1,00 g
- Triéthanolamine (99 %)........... 1,00 g
- Lauryl sulfate de sodium......... 0,10 g
- Eau purifiée..................... 75,05 g
- Peroxyde de benzoyle hydraté à 25 %.......................... 12,80 g

Le mélange des deux gels se fait extemporanément, poids pour poids au moment de l'emploi.

On applique ce mélange 2 fois par jour sur une peau acnéique. Après 4 à 6 semaines de traitement à raison de 2 à 10 mg par $cm^2$ de peau traitée et par application, on observe une amélioration significative.

## Revendications

1. Composé ayant la formule générale :

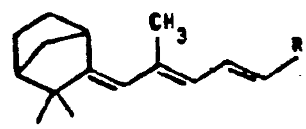

(I)

ou sel correspondant, formule dans laquelle R représente soit un groupement de formule :

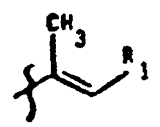

(II)

soit un groupement de formule :

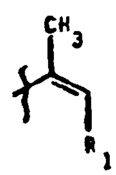

(III)

$R_1$ représentant un radical pris dans le groupe formé par les radicaux :

. oxazolinyle
. $-C\equiv N$
. $-CH_2-O-R_2$
. $-\underset{\underset{O}{\|}}{C}-R_3$

– $R_2$ représentant un atome d'hydrogène, un radical alkyle en $C_1$-$C_6$, un radical cyclopentyle, un radical cyclohexyle, un radical tétrahydropyrannyle, un radical aralkyle en $C_7$-$C_9$ éventuellement substitué par un ou plusieurs groupements alcoxy en $C_1$-$C_6$, un radical de formule :

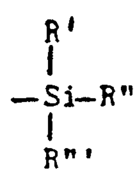

(IV)

dans laquelle R′, R″ et R‴, identiques ou différents, représentent un reste alkyle, linéaire ou ramifié, en $C_1$-$C_6$ ou un reste aryle éventuellement substitué par un radical alkyle en $C_1$-$C_4$

– $R_3$ représentant un atome d'hydrogène, le radical $-O-R_4$ ou le radical

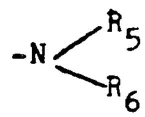 ,

$R_5$ et $R_6$, identiques ou différents, représentant un atome d'hydrogène, un radical alkyle en $C_1$-$C_6$ ou, pris ensemble, formant un hétérocycle en $C_5$-$C_8$, $R_4$ représentant un atome d'hydrogène ou un radical alkyle en $C_1$-$C_6$.

2. Composé selon la revendication 1 correspondant à la formule :

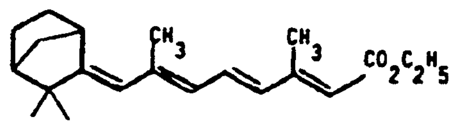

($I_A$) .

3. Composé selon la revendication 1 correspondant à la formule :

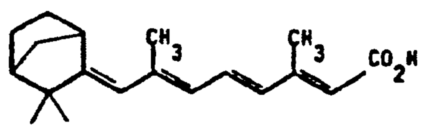

($I_B$)

4. Composé selon la revendication 1 correspondant à la formule :

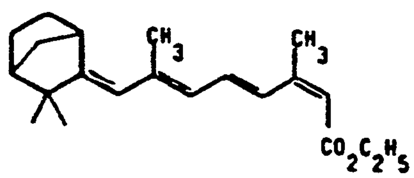

($I_C$)

5. Composé selon la revendication 1 correspondant à la formule :

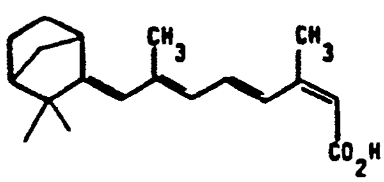

($I_D$)

6. Procédé de fabrication permettant d'obtenir les composés selon l'une des revendications 1 à 5, caractérisé par le fait que, dans une première étape, l'on oxyde le dl-patchénol de formule (V) :

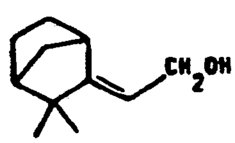

(V)

en dl-patchénal de formule (VI) :

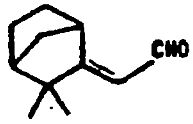

(VI)

par $MnO_2$ activé en milieu solvant ; dans une deuxième étape, on fait agir sur le composé de formule (VI) une solution d'iodure de méthylmagnésium en milieu solvant et, après avoir stoppé la réaction, on oxyde lentement la solution par $MnO_2$ pour obtenir le composé de formule (VII) :

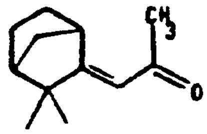

(VII)

dans une troisième étape, on prépare l'organomagnésien H-C≡C-MgBr en milieu solvant et on le fait agir sous courant d'acétylène,sur le composé de formule (VII) pour obtenir le composé de formule (VIII) :

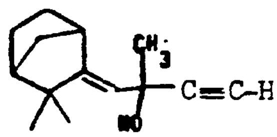

(VIII)

dans une quatrième étape, on fait agir en milieu solvant le bromure d'éthylmagnésium sur le composé de formule (VIII) puis on ajoute dans la solution du trans- ou du cis-formyl-3-butène-2-oate d'alkyl inférieur selon que l'on désire un composé de formule (I) dans lequel R représente un groupement de formule (II) ou (III) respectivement, afin d'obtenir respectivement un composé de formule (IX) ou (XVI) :

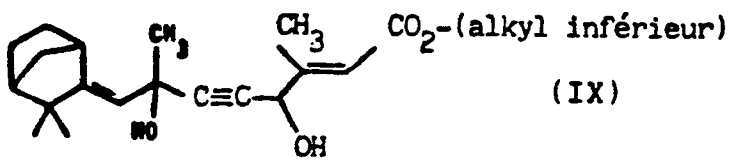

(IX)

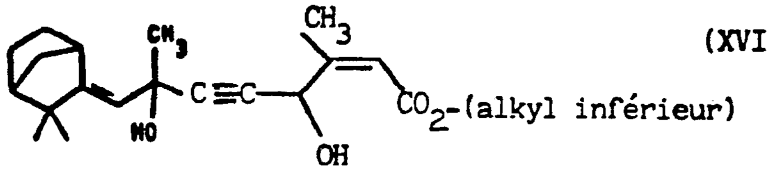

(XVI)

dans une cinquième étape, on fait agir, en milieu solvant, sur le composé ainsi obtenu, de l'hydrure de diisobutylaluminium pour obtenir le triol de formule (X) ou (XVII) respectivement :

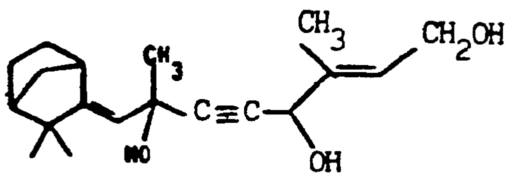

(X)

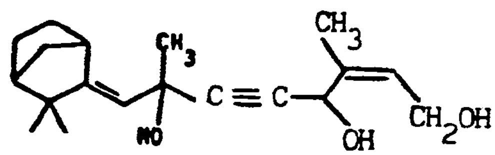

(XVII)

dans une sixième étape, on bloque la fonction OH en bout de chaine du triol obtenu en faisant agir sur ce composé un halogénure de tri-(alkyl ou aryl) silyle en présence d'imidazole pour obtenir le composé de formule (XI) ou (XVIII) respectivement :

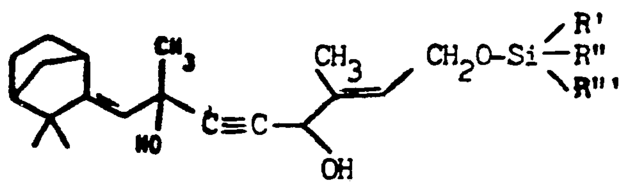

(XI)

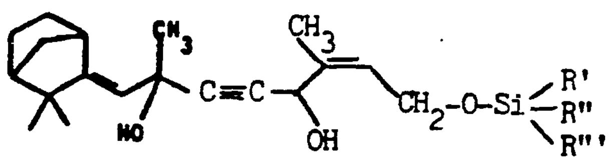

(XVIII)

ou R', R", R"' ont les significations précédemment indiquées, on réduit ce dernier composé en milieu solvant par l'hydrogène en présence d'un catalyseur Pd/$CaCO_3$ désactivé au plomb pour obtenir le diol de formule (XII) ou (XIX) respectivement :

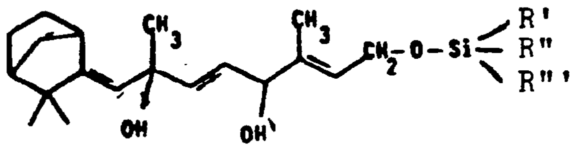

(XII)

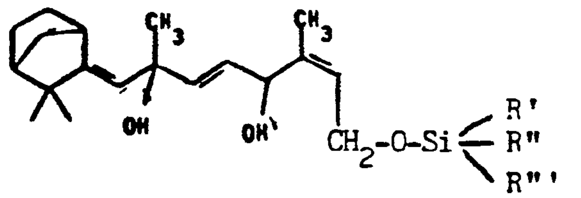

(XIX)

puis on élimine les deux fonctions OH du diol en faisant agir sur le composé obtenu, en milieu solvant anhydre, un mélange (trichlorure de titane, hydrure d'aluminium et de lithium) pour obtenir le composé de formule (XIII) ou (XX) respectivement :

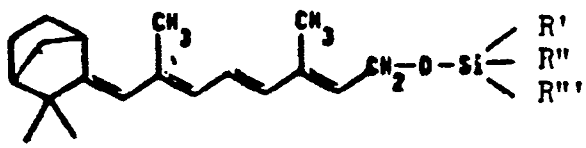

(XIII)

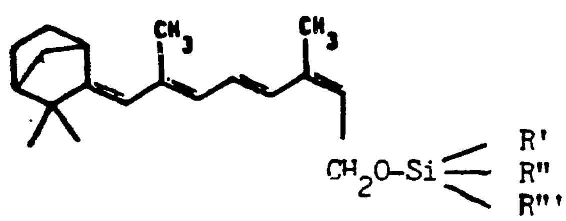

(XX)

et, dans une éventuelle septième étape, on transforme ce dernier composé pour obtenir un autre composé de formule (I) où R est un groupement de formule (II) ou (III) dans lequel $R_1$ est différent de

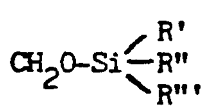

.

7. Procédé selon la revendication 6, destiné à préparer un composé dans lequel $R_1$ est un radical $CH_2OH$, caractérisé par le fait que dans une septième étape on débloque l'extrémité de la chaîne du composé de formule (XIII) ou (XX) par action en milieu solvant, d'au moins un fluorure pour obtenir le composé de formule (XIV) ou (XXI) respectivement :

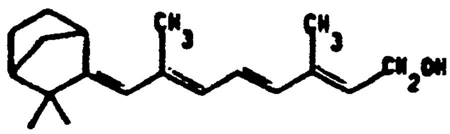

(XIV)

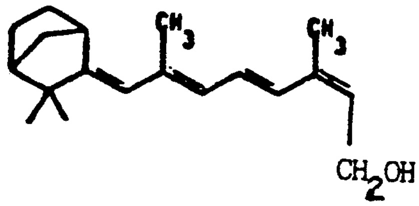

(XXI)

8. Procédé selon la revendication 6, destiné à préparer un composé dans lequel $R_1$ est un radical CHO, caractérisé par le fait que, dans une septième étape, on agit sur le composé de formule (XIII) ou (XX) comme indiqué dans la revendication 7, et que l'on oxyde le composé de formule (XIV) ou (XXI) en milieu solvant anhydre par action de $MnO_2$.

9. Procédé selon la revendication 6, destiné à préparer un composé dans lequel $R_1$ est un radical $CO_2C_2H_5$, caractérisé par le fait que, dans une septième étape, on agit sur le composé de formule (XIII) ou (XX) comme indiqué à la revendication 8, et que l'on soumet le produit obtenu dissous dans l'éthanol à l'action d'un cyanure alcalin et de l'oxyde d'argent.

10. Procédé selon la revendication 6, destiné à préparer un composé dans lequel $R_1$ est un radical $CO_2H$, caractérisé par le fait que, dans une septième étape, l'on agit sur le composé de formule (XIII) ou (XX) comme indiqué à la revendication 9, et que l'on soumet l'ester obtenu à l'action d'une solution hydroalcoolique d'une base forte et que l'on ramène ensuite la solution à un pH acide.

11. Composition médicamenteuse, caractérisée par le fait qu'elle comporte, dans un support pharmaceutiquement acceptable, au moins un composé de formule (I) ou un sel correspondant.

12. Composition selon la revendication 11, caractérisée par le fait qu'elle est destinée à traiter les affections dermatologiques liées à un désordre de la kératinisation, les affections dermatologiques à composantes inflammatoires et/ou immunoallergiques et, notamment, les acnés, le psoriasis, les troubles de la kératinisation les ichtyoses, la maladie de Darier, les kératodermies palmo-plantaires, les leucoplasies, le lichen plan, toutes proliférations dermatologiques bénignes ou malignes, le psoriasis rhumatoïde, les atopies cutanées ou respiratoires, ainsi que certains problèmes ophtalmologiques relatifs aux cornéopathies.

13. Composition selon l'une des revendications 11 ou 12, caractérisée par le fait que, pour l'administration par voie topique, elle renferme une concentration en composé(s) de formule (I) allant de 0,0001 à 5% en poids et, de préférence, comprise entre 0,001 et 1% en poids par rapport au poids total de la composition.

14. Composition cosmétique, caractérisée par le fait qu'elle contient, dans un support cosmétiquement acceptable, au moins un composé de formule (I) ou un sel correspondant.

15. Composition selon la revendication 14, caractérisée par le fait qu'elle se présente sous forme de lotion, de gel, de savon ou de shampooing.

16. Composition selon l'une des revendications 14 ou 15, caractérisée par le fait que sa concentration en composé(s) de formule (I) est comprise entre 0,0001 et 2% et, de préférence, entre 0,001 et 1% en poids par rapport au poids total de la composition.

17. Composition selon l'une des revendications 11 à 16, caractérisée par le fait qu'elle contient au moins un additif, inerte ou actif, choisi dans le groupe formé par des agents hydratants, des agents anti-séborrhéiques, des agents anti-acnéiques, des antibiotiques, des agents favorisant la repousse des cheveux, des agents anti-inflammatoires, des caroténoïdes et des agents anti-psoriasiques.

**Ansprüche**

1. Verbindungen der allgemeinen Formel :

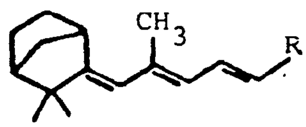

(I)

oder die Salze davon, worin R eine Gruppe der allgemeinen Formel :

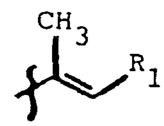

(II)

oder eine Gruppe der allgemeinen Formel :

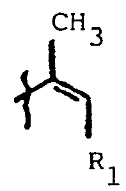

(III)

bedeutet, worin $R_1$ ein Rest darstellt, ausgewählt unter :

- Oxazolinyl
- $-C \equiv N$
- $-CH_2-O-R_2$
- 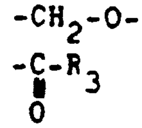

– $R_2$ ein Wasserstoffatom, einen $C_1$-$C_6$-Alkylrest, einen Cyclopentylrest, einen Cyclohexylrest, einen Tetrahydropyranylrest, einen gegebenenfalls durch eine oder mehrere $C_1$-$C_6$-Alkoxygruppen substituierten $C_7$-$C_9$-Aralkylrest, einen Rest der allgemeinen Formel :

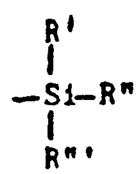

(IV)

worin R′, R″ und R‴ gleich oder verschieden sind und einen geradkettigen oder verzweigten $C_1$-$C_6$-Alkylrest bedeuten, oder einen gegebenenfalls durch einen $C_1$-$C_4$-Alkylrest substituierten Arylrest bedeutet ;

– $R_3$ ein Wasserstoffatom, einen Rest $-O-R_4$ oder einen Rest

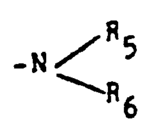

bedeutet, wobei $R_5$ und $R_6$ gleich oder verschieden sind und ein Wasserstoffatom, einen $C_1$-$C_6$-Alkylrest bedeuten oder zusammen einen $C_5$-$C_8$-Heterozyklus bilden, $R_4$ ein Wasserstoffatom oder ein $C_1$-$C_6$-Alkylrest bedeutet.

2. Verbindung nach Anspruch 1, der Formel :

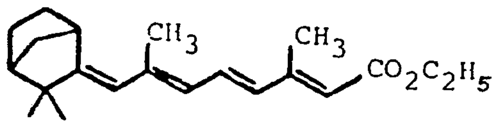

$(I_A)$

3. Verbindung nach Anspruch 1, der Formel :

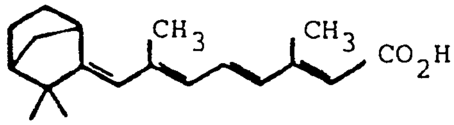

$(I_B)$

4. Verbindung nach Anspruch 1, der Formel :

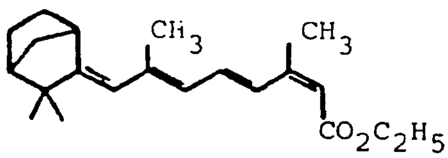

$(I_C)$

5. Verbindung nach Anspruch 1, der allgemeinen Formel :

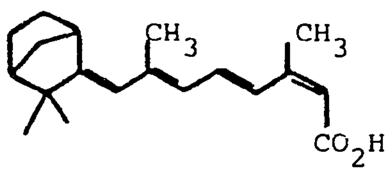

$(I_D)$

6. Verfahren zur Herstellung von Verbindungen nach einem der Ansprüche 1 bis 5, dadurch gekennzeichnet, daß man in einem ersten Schritt d, l-Patchenol der Formel (V) :

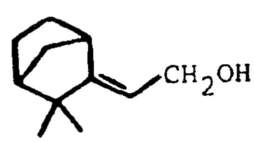

(V)

zu d, l-Patchenal der Formel (VI) :

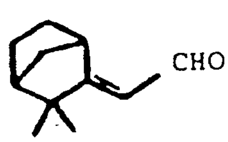

(VI)

mit aktiviertem $MnO_2$ in einem Lösungsmittel oxydiert ; in einem zweiten Schritt die Verbindung der Formel (VI) mit einer Methylmagnesiumjodidlösung in einem Lösungsmittel reagieren läßt und nach Abstoppen der Reaktion die Lösung mit $MnO_2$ langsam oxydiert, wobei man eine Verbindung der Formel VII :

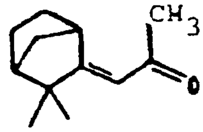

(VII)

erhält ;

in einem dritten Schritt die Organomagnesiumverbindung H-C=C-MgBr in Lösung herstellt und unter einem Acetylenstrom mit der Verbindung der Formel (VII) reagieren läßt, wobei man die Verbindung der Formel (VIII):

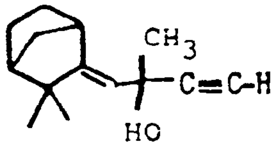

(VIII)

erhält ;

in einem vierten Schritt Ethylmagnesiumbromid in Lösung mit der Verbindung der Formel (VIII) umsetzt und anschließend zur Lösung, je nach dem ob man eine Verbindung der Formel (I) wünscht, worin R eine Gruppe der Formel (II) oder (III) darstellt, Niedrigalkyl- trans- oder cis-formyl-3-buten-2-oat hinzugibt, um eine Verbindung der Formel (IX) bzw. (XVI) zu erhalten :

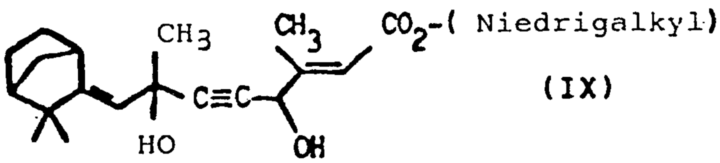

(IX)

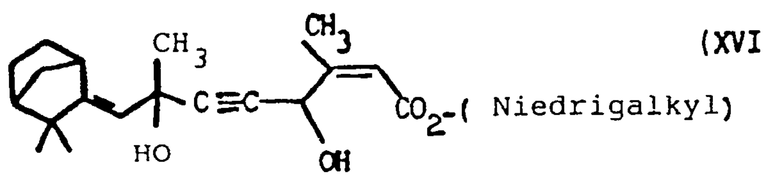

(XVI)

in einem fünften Schritt die so erhaltene Verbindung in Lösung mit Diisobutylaluminiumhydrid reagieren läßt, wobei man das Triol der allgemeinen Formel (X) bzw. (XVII) erhält :

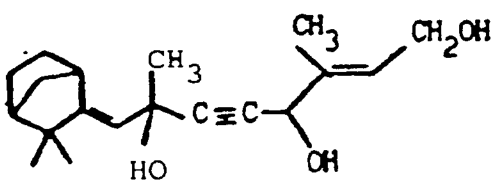

(X)

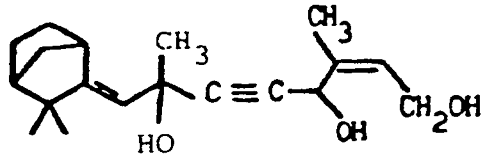

(XVII)

in einem sechsten Schritt die OH-Funktion am Ende der erhaltenen Triolkette durch Behandlung dieser Verbindung mit einem Tri-(alkyl oder aryl)silylhalogenid in Gegenwart von Imidazol blockiert, wobei man eine Verbindung der Formel (XI) bzw. (XVIII) erhält :

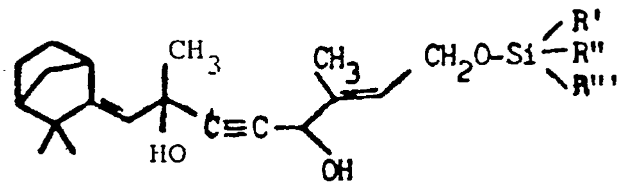

(XI)

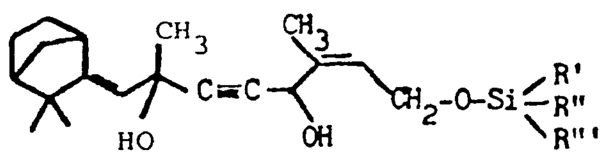

(XVIII)

worin R′, R″ und R‴ die oben angegebenen Bedeutungen besitzen, diese Verbindung in Lösung mit Wasserstoff in Gegenwart eines mit Blei desaktivierten $Pd/CaCO_3$ Katalysators zum Diol der allgemeinen Formel (XII) bzw. (XIX) reduziert :

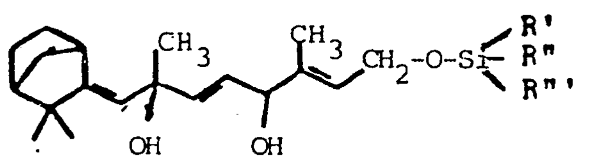

(XII)

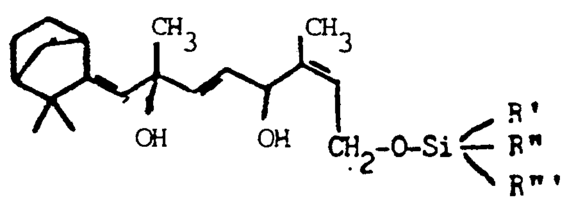

(XIX)

anschließend die beiden OH-Funktionen des Diols eliminiert, indem man die erhaltene Verbindung in wasserfreier Lösung mit einem Gemisch (Trichlortitan, Lithiumaluminiumhydrid) behandelt, wobei man eine Verbindung der allgemeinen Formel (XIII) bzw. (XX) erhält :

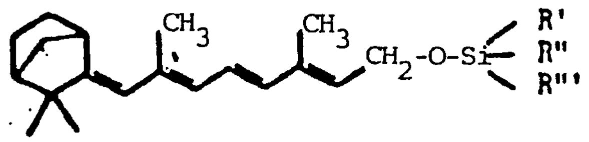

(XIII)

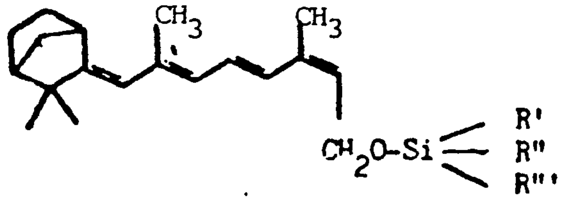

(XX)

und gegebenenfalls in einem siebten Schritt diese Verbindung in eine andere Verbindung der allgemeinen Formel (I), worin R eine Gruppe der allgemeinen Formel (II) oder (III) darstellt, worin $R_1$ von

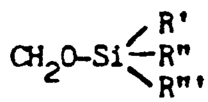

verschieden ist, überführt.

7. Verfahren nach Anspruch 6, zur Herstellung einer Verbindung, worin $R_1$ einen $CH_2OH$-Rest bedeutet, dadurch gekennzeichnet, daß man in einem siebten Schritt das Kettenende der Verbindung der allgemeinen Formel (XIII) oder (XX) durch Behandlung mit mindestens einem Fluorid in einem Lösungsmittel deblockiert, wobei man eine Verbindung der Formel (XIV) oder (XXI) erhält :

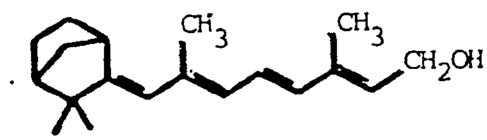

(XIV)

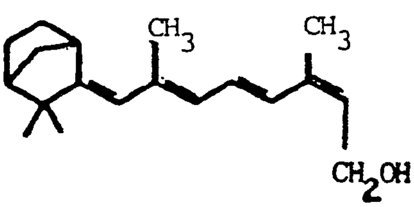

(XXI)

8. Verfahren nach Anspruch 6, zur Herstellung einer Verbindung worin $R_1$ einen CHO-Rest bedeutet, dadurch gekennzeichnet, daß man in einem siebten Schritt die Verbindung der allgemeinen Formel (XIII) oder (XX) gemäß Anspruch 7 behandelt und man die Verbindung der Formel (XIV) bzw. (XXI) in einem wasserfreien Lösungsmittel mit $MnO_2$ oxydiert.

9. Verfahren nach Anspruch 6, zur Herstellung einer Verbindung, worin $R_1$ einen $CO_2C_2H_5$-Rest bedeutet, dadurch gekennzeichnet, daß man in einem siebten Schritt eine Verbindung der allgemeinen Formel (XIII) oder (XX) gemäß Anspruch 8 behandelt und man das erhaltene Produkt, gelöst in Ethanol mit einem Alkalicyanid und Silberoxyd umsetzt.

10. Verfahren nach Anspruch 6, zur Herstellung einer Verbindung, worin $R_1$ einen $CO_2H$-Rest bedeutet, dadurch gekennzeichnet, daß man in einem siebten Schritt eine Verbindung der allgemeinen Formel (XIII) oder (XX) wie in Anspruch 9 behandelt und man den erhaltenen Ester in einer wässrigalkoholischen Lösung mit einer starken Base umsetzt und die Lösung anschließend auf einen sauren pH bringt.

11. Arzneimittel, dadurch gekennzeichnet, daß es in einem pharmazeutisch verträglichen Träger mindestens eine Verbindung der allgemeinen Formel I oder ein Salz davon umfaßt.

12. Mittel nach Anspruch 11, dadurch gekennzeichnet, daß es bestimmt ist zur Behandlung von dermatologischen Erkrankungen, die mit einer Störung der Keratinisierung verbunden sind, dermatologischen Erkrankungen mit entzündlicher und/oder immunoallergischer Komponente und, insbesondere von Akne, Psoriasis, Keratinisierungsstörungen, Ichtyosen, Darier-Krankheit, palmoplantaren Keratodermien, Leukoplasien, Lichen planus, allen benignen und malignen Poliferationsdermatologien, rheumatischer Psoriasis, cutanen oder respira-torischen Atopien, sowie von bestimmten ophthalmologischen Störungen aufgrund von Corneopathien.

13. Mittel nach einem der Ansprüche 11 oder 12, dadurch gekennzeichnet, daß es zur topischen Verabreichung eine Konzentration der Verbindung(en) der allgemeinen Formel (I) von 0,0001 bis 5 Gew.-% und vorzugsweise von 0,001 bis 1 Gew.-%, bezogen auf das Gesamtgewicht des Mittels, aufweist.

14. Kosmetisches Mittel, dadurch gekennzeichnet, daß es in einem kosmetisch verträglichen Träger mindestens eine Verbindung der allgemeinen Formel (I) oder ein Salz davon enthält.

15. Mittel nach Anspruch 14, dadurch gekennzeichnet, daß es als Lotion, als Gel, als Seife oder als Shampoo vorliegt.

16. Mittel nach einem der Ansprüche 14 oder 15, dadurch gekennzeichnet, daß die Konzentration der Verbindung(en) der allgemeinen Formel (I) im Bereich von 0,0001 bis 2% und vorzugsweise zwischen 0,001 und 1 Gew.-%, bezogen auf das Gesamtgewicht des Mittels, liegt.

17. Mittel nach einem der Ansprüche 11 bis 16, dadurch gekennzeichnet, daß es mindestens ein inertes oder aktives Additiv enthält, das ausgewählt ist unter Hydratisierungsmitteln, Antiseborrhoemitteln, Antiaknemitteln, Antibiotika, Mitteln zur Förderung des Haarwuchses, antiinflammatorischen Mitteln, Carotinoiden und Antipsoriasismitteln.

**Claims**

1. Compound having the general formula :

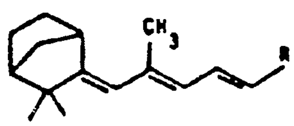

(I)

or a corresponding salt, in which formula R represents either a group of formula :

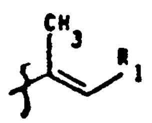

(II)

or a group of formula :

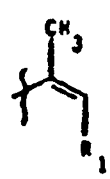

(III)

$R_1$ representing a radical selected from the group consisting of the radicals :

- oxazolinyl
- $-C\equiv N$
- $-CH_2-O-R_2$
- $-\underset{\underset{O}{\|}}{C}-R_3$

$-R_2$ representing a hydrogen atom, a $C_1$-$C_6$-alkyl radical, a cyclopentyl radical, a cyclohexyl radical, a tetrahydropyranyl radical, a $C_7$-$C_9$-aralkyl radical optionally substituted with one or more $C_1$-$C_6$-alkoxy groups or a radical of formula :

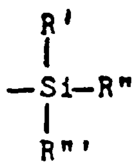

(IV)

in which R′, R″ and R‴, which may be identical or different, represent a linear or branched $C_1$-$C_6$-alkyl residue or an aryl residue optionally substituted with a $C_1$-$C_4$-alkyl radical,
$-R_3$ representing a hydrogen atom, the radical $-O-R_4$ or the radical

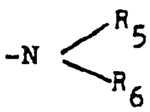 ,

$R_5$ and $R_6$, which may be identical or different, representing a hydrogen atom or a $C_1$-$C_6$-alkyl radical or, taken together, forming a $C_5$-$C_8$-heterocycle,
$R_4$ representing a hydrogen atom or a $C_1$-$C_6$-alkyl radical.

2. Compound according to Claim 1, corresponding to formula :

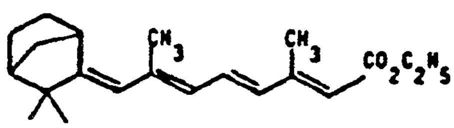

$(I_A)$

3. Compound according to Claim 1, corresponding to formula :

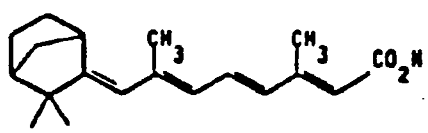

$(I_B)$

4. Compound according to Claim 1, corresponding to formula :

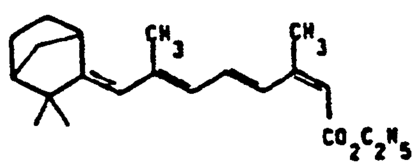

$(I_C)$

5. Compound according to Claim 1, corresponding to formula :

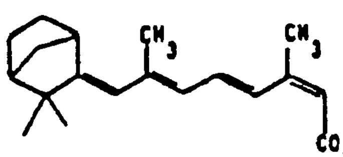

$(I_D)$

6. Manufacturing process enabling the compounds according to one of Claims 1 to 5 to be obtained, characterised in that, in a first step, dl-patchenol of formula (V) :

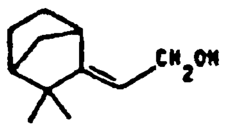

(V)

is oxidised to dl-patchenal of formula (VI) :

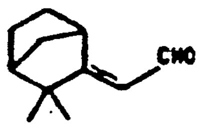

(VI)

with activated $MnO_2$ in a solvent medium ; in a second step, a solution of methylmagnesium iodide in a solvent medium is reacted with the compound of formula (VI) and, after the reaction has been stopped, the solution is oxidised slowly with MnO2 to obtain the compound of formula (VII) :

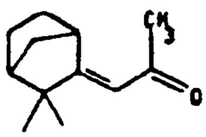

(VII)

in a third step, the organomagnesium compound H-C≡C-MgBr is prepared in a solvent medium and reacted in a stream of acetylene with the compound of formula (VII) to obtain the compound of formula (VIII) :

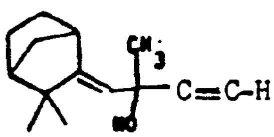

(VIII)

in a fourth step, ethylmagnesium bromide is reacted in a solvent medium with the compound of formula (VIII), and a lower alkyl trans- or cis-formyl-3-buten-2-oate, depending on whether a compound of formula (I) in which R represents a group of formula (II) or (III), respectively, is desired, is then added to the solution in order to obtain, respectively, a compound of formula (IX) or (XVI) :

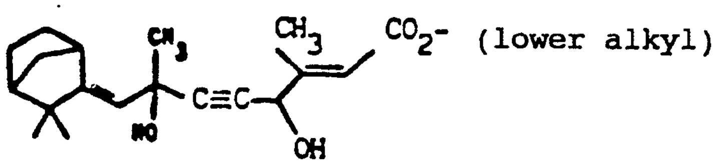

(IX)

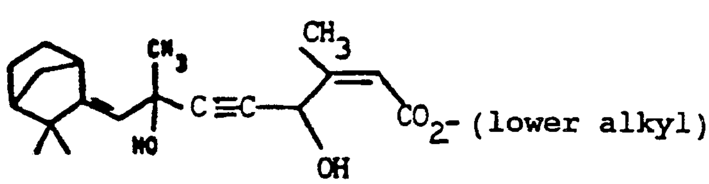

(XVII)

in a fifth step, the compound thereby obtained is reacted in a solvent medium with diisobutylaluminium hydride

to obtain the triol of formula (X) or (XVII), respectively :

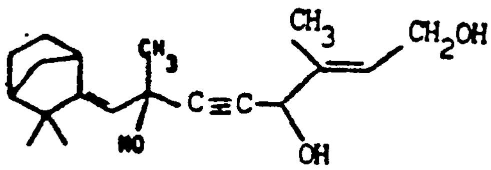

(X)

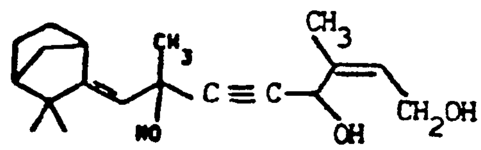

(XVII)

in a sixth step, the chain-end OH function of the triol obtained is blocked by reacting this compound with a tri-(alkyl or aryl)silyl halide in the presence of imidazole to obtain the compound of formula (XI) or (XVIII), respectively :

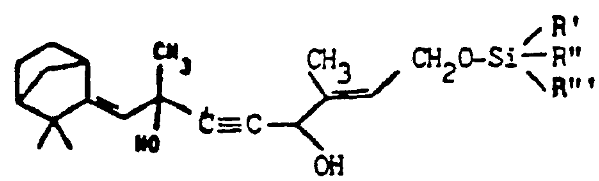

(XI)

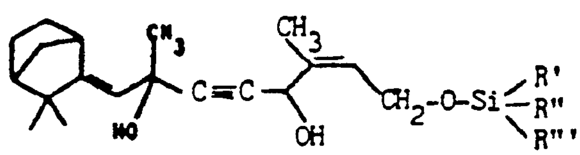

(XVIII)

where R′, R″ and R‴ have the meanings stated above, the latter compound is reduced in a solvent medium with hydrogen in the presence of a lead-deactivated $Pd/CaCO_3$ catalyst to obtain the diol of formula (XII) or (XIX), respectively :

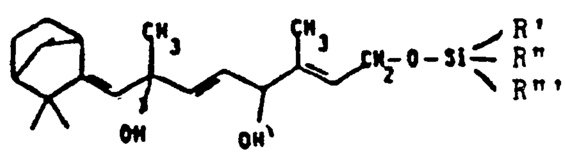

(XII)

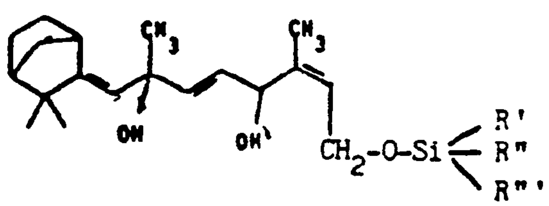

(XIX)

the two OH functions of the diol are then removed by reacting the compound obtained with a titanium trichloride/lithium aluminium hydride mixture in an anhydrous solvent medium to obtain the compound of formula (XIII) or (XX), respectively :

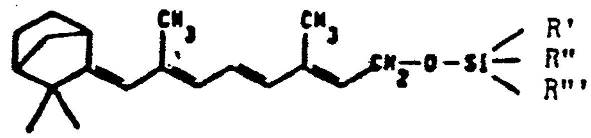

(XIII)

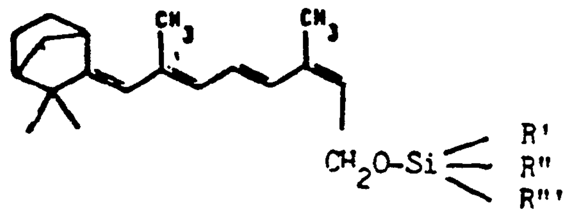

(XX)

and, in an optional seventh step, the latter compound is converted to obtain another compound of formula (I) wherein R is a group of formula (II) or (III) in which $R_1$ is other than

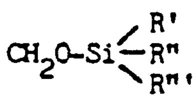 .

7. Process according to Claim 6, intended for preparing a compound in which $R_1$ is a $CH_2OH$ radical, characterised in that, in a seventh step, the end of the chain of the compound of formula (XIII) or (XX) is unblocked by the action of at least one fluoride in a solvent medium to obtain the compound of formula (XIV) or (XXI), respectively :

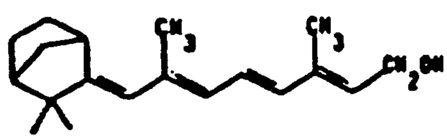

(XIV)

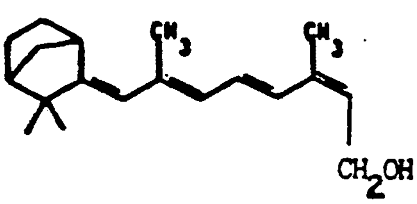

(XXI)

8. Process according to Claim 6, intended for preparing a compound in which $R_1$ is a CHO radical, characterised in that, in a seventh step, the compound of formula (XIII) or (XX) is treated as described in Claim 7, and in that the compound of formula (XIV) or (XXI) is oxidised in an anhydrous solvent medium by the action of $MnO_2$.

9. Process according to Claim 6, intended for preparing a compound in which $R_1$ is a $CO_2C_2H_5$ radical, characterised in that, in a seventh step, the compound of formula (XIII) or (XX) is treated as described in Claim 8, and in that the product obtained, dissolved in ethanol, is subjected to the action of an alkali metal cyanide and silver oxide.

10. Process according to Claim 6, intended for preparing a compound in which $R_1$ is a $CO_2H$ radical, characterised in that, in a seventh step, the compound of formula (XIII) or (XX) is treated as described in Claim 9, in that the ester obtained is subjected to the action of an aqueous-alcoholic solution of a strong base and in that the solution is then readjusted to an acid pH value.

11. Medicinal composition, characterised in that it contains at least one compound of formula (I) or a corresponding salt in a pharmaceutically acceptable vehicle.

12. Composition according to Claim 11, characterised in that it is intended for treating dermatological conditions linked to a disorder of keratinisation, dermatological conditions having inflammatory and/or immunoallergic components, and in particular acne, psoriasis, disorders of keratinisation, ichthyoses, Darier's disease, palmoplantar keratoderma, leukoplakia, lichen planus, all benign or malignant dermatological proliferations, rheumatoid psoriasis, cutaneous or respiratory atopy and also some ophthalmological problems relating to corneopathies.

13. Composition according to one of Claims 11 and 12, characterised in that, for topical administration, it contains a concentration of compound(s) of formula (I) ranging from 0.0001 to 5% by weight, and preferably between 0.001 and 1% by weight, relative to the total weight of the composition.

14. Cosmetic composition, characterised in that it contains at least one compound of formula (I) or a corresponding salt in a cosmetically acceptable vehicle.

15. Composition according to Claim 14, characterised in that it takes the form of a lotion, gel, soap or shampoo.

16. Composition according to one of Claims 14 and 15, characterised in that the concentration of compound(s) of formula (I) therein is between 0.0001 and 2%, and preferably between 0.001 and 1%, by weight relative to the total weight of the composition.

17. Composition according to one of Claims 11 to 16, characterised in that it contains at least one inert or active additive selected from the group consisting of hydrating agents, antiseborrheic agents, anti-acne agents, antibiotics, agents promoting hair regrowth, anti-inflammatory agents, carotenoids and antipsoriatic agents.